# EUROPEAN PATENT APPLICATION

(11) **EP 2 000 477 A1**
(43) Date of publication of application: **10.12.2008**
(21) Application number: 07011200.8
(22) Date of filing: 07.06.2007
(51) Int. Cl.: C07K 14/32, C12N 15/75

(54) **Increased production of a target product via stabilization of mRNA**

(71) Applicant: DSM IP Assets B.V., 6411 TE Heerlen (NL)
(72) Inventor: The designation of the inventor has not yet been filed
(74) Representative: Seibel-Thomsen, Nadja

(57) **Abstract**

The present invention relates to newly identified mRNA stabilizing elements useful for the production of a target fennentation product, such as e.g. vitamins or enzymes, in particular riboflavin (vitamin B2), biotin, pantothenic acid (vitamin B5), folic acad, thiamin, pyridoxin (vitamin B6), vitamin B12, xylanase, amylase, protease, glucarase, amylomaltase or maltogenic amylase.

## Description

The present invention relates to newly identified mRNA stabilizing elements useful for the production of a target fermentation product, such as e.g. vitamins or enzymes, in particular riboflavin (vitamin B2), biotin, pantothenic acid (vitamin B5), folic acid, thiamin, pyridoxin (vitamin B6), vitamin B12, xylanase, amylase, protease, glucanase, amylomaltase or maltogenic amylase.

Many commercially valuable products are produced by fermentation reactions, such as e.g. vitamins or enzymes.

Several methods are available for the production of target fermentation products, including e.g. replacing the natural (weak) promoter by a strong promoter or amplification of expression cassettes within the chromosome, said cassette containing a single promoter operably linked to a gene of interest and an amplifiable selectable marker gene, e.g. an antibiotic resistance marker. The amplification leads to the production of multiple copies of the expression cassette and the selectable marker gene in the chromosome. One could also increase the amount of a desired fermentation product by decoupling the production of the desired product from the growth of said host cell.

However, there are disadvantages associated with the above-mentioned approaches. For example, it may not be possible to achieve saturating levels of mRNA by amplification of genes driven by a single promoter. Furthermore, the production of multiple copies of the expression cassette and the selectable marker gene in the chromosome of a host cell may not be stable or might even prevent further expression of the respective gene (feedback inhibition).

It is an object of the present invention to improve the yield and/or productivity of a target fermentation product, in particular the production of vitamins or enzymes.

Surprisingly, it has now been found that the production of a target fermentation product could be enhanced by increasing the transcript stability, *i.e.* stabilization of mRNA generated via transcription of the respective gene(s) involved in the biosynthesis of such target fermentation products.

In particular, it has now been found that the introduction of a polynucleotide having a nucleotide sequence that hybridizes preferably under highly stringent conditions to a sequence shown in SEQ ID NO:1 to 5 plays an important role in stabilization of mRNA transcribed from a respective gene. The mRNA stabilizing element may be introduced downstream of the transcription start of a respective target gene. It has also been found that by introducing said polynucleotide or mRNA stabilizing clement into a suitable microorganism, such as for example *Bacillus,* the production of a target fermentation product can be greatly improved due to stabilized mRNA transcripted from the respective gene(s) involved in production of the desired target product.

The target fermentation products may be selected from vitamins or enzymes. In the case of vitamins, the target fermentation product is particularly selected from the group consisting of riboflavin (vitamin B2), biotin, pantothenic acid (vitamin B5), folic acid, thiamin and pyridoxin (vitamin B6). In the case of enzymes, the target fermentation product is particularly selected from the group consisting of transferases [EC 2] and hydrolases [EC 3], preferably selected from glycosyltransferases [EC 2,4], glycosylases [EC 3.2] or peptidases [EC 3.4], more preferably selected from glycosidases [EC 3.2.1] or hexosyltranferases [EC 2.4.1], such as e.g. α-amylase, xylanase, β-glucanase, maltogenic amylase (glucan 1,4-α-maltohydrolase), neutral protease/proteinase or amylomaltase (4-α-glucanotransferase).

In one embodiment, the present invention is directed to fermentative production of riboflavin (vitamin B2). As used herein, the term "riboflavin" includes but is not limited to riboflavin, flavin mononucleotide (FMN), flavin adenine dinucleotide (FAD), as well as precursors, derivatives and salts of riboflavin, FMN or FAD, such as e.g. riboflavin-5-phosphate or sodium riboflavin-5-phosphate. Precursors and/or derivatives of riboflavin, FMN and FAD may be selected from *e.g.* DRAPP; 5-amino-6-ribosylamuino-2,4 (1H,3H)-pyrimidinedione-5'-phosphate, 2,5-diamino-6-ribitylamino-4 (3H)-pyrimidinone-5'-phosphate; 5-amino-6-ribitylamino-2,4 (1H,3H)-pyrimidinedione-5'-phosphate; 5-amino-6-ribitylamino-2,4 (1H,3H)-pyrimidinedione; 6,7-dimethyl-8-ribityllumazine (DMRL); and flavoproteins. The terms "riboflavin" and "vitamin B2" are used interchangeably herein. The genes involved in biosynthesis of riboflavin as well as methods for fermentative production of riboflavin are known (see e.g. EP 405370 or Ullman's Encyclopedia of Industrial Chemistry, 7th Edition, 2007, Chapter Vitamins). These methods may be also applied for production of riboflavin using an mRNA stabilizing element as described herein.

The *B. subtilis* riboflavin *(rib)* operon consists of the medium strong *P_{rib}* promoter, a 5' leader sequence containing a so-called riboswitch and the genes *ribD (ribG), ribE (ribB), ribA, ribH,* and *ribT,* Beside RibT, the function and catalytic activity of all gene products is known. The riboswitch of the 5' leader sequence is able to bind flavin monophosphate (FMN). After FMN binding a refolding of the leader prevents further transcription of the five genes in the *rib* operon. In the absence of FMN the structure of the riboswitch allows the transcription of the five *rib* genes and consequently the synthesis of riboflavin.

In one embodiment, the present invention is directed to fermentative production of biotin. As used herein, the term "biotin" includes but is not limited to biotin, precursors and/or derivatives of biotin. Examples of such precursors are selected from pimelic acid, pimelyl-CoA, 7-keto-8-amino-pelargonic acid (7-KAP), 7,8-diamino-pelargonic acid (DAPA) or dethiobiotin (DTB). The genes involved in biosynthesis of biotin as well as methods for fermentative production of biotin are known (see *e.g.* EP 635572 or EP 892066 or Ullman's Encyclopedia of Industrial Chemistry, 7th Edition, 2007, Chapter Vitamins). These methods may be also applied for production of biotin using an mRNA stabilizing element as described herein.

In a further embodiment, the target fermentation product is pantothenic acid (vitamin B5). As used herein, the term "pantothenic acid" includes but is not limited to pantothenic acid, precursors and/or derivatives thereof such as salts or esters thereof, *i.e.* pantothenate, in particular calcium pantothenate, or the alcohol form of pantothenic acid, *i.e.* pantothenol or panthenol. The terms "pantothenic acid", "pantothenate" and "vitamin B5" are used interchangeably herein. Precursors/intermediates in the biosynthetic pathway of pantothenic acid which are included may be selected from *e.g.* pantoate, α-ketopantoate or α-ketoisovalerate. The genes involved in biosynthesis of pantothenic acid as well as methods for fermentative production of pantothenic acid are known (see *e.g.* WO 01/21772, WO 02/057474, WO 02/061108 or Ullman's Encyclopedia of Industrial Chemistry, 7th Edition, 2007, Chapter Vitamins). These methods may be also applied for production of pantothenic acid using an mRNA stabilizing element as described herein.

In another embodiment, the target fermentation product is folic acid. As used herein, the term "folic acid" includes but is not limited to folic acid and precursors/derivatives thereof, such as *e.g.* folate, guanosine 5'-triphosphate (GTP), p-ammobenzoic acid, L-glutamic acid, dihydroneopterin tri-/mono-phosphate, dihydroneopterin, hydroxymethyldihydropterin, hydroxymethyldihydropterin pyrophosphate, dihydropteric acid, and dihydrofolic acid. The genes involved in biosynthesis of folic acid as well as methods for fermentative production of folic acid are known (see e.g. Ullman's Encyclopedia of Industrial Chemistry, 7th Edition, 2007, Chapter Vitamins). These methods may be also applied for production of folic acid using an mRNA stabilizing element as described herein,

In a further embodiment, the target fermentation product is thiamin. As used herein, the term "thiamin" includes but is not limited to thiamin, thiamin monophosphate (TMP), thiamin pyrophosphate and precursors/derivatives thereof, such as, e.g. 4-amino-5-hydroxymethyl-2-methylpyimidine (HMP), 5-(2-hydroxyethyl)-4-methylthiazole (HET) and a combination thereof. Furthermore included are precursors and/or derivatives of the HMP and/or HET-pathway, such as e.g. glycine, cysteine, isoleucine, threonine, 5-aminoimidazole ribotide (AIR) or 4-amino-2-methyl-5-pyrimidinemethaneamine (Grewe Diamine). The genes involved in biosynthesis of thiamin as well as methods for fermentative production of thiamin are known (see *e.g.* WO 2004/106557 or Ullman's Encyclopedia of Industrial Chemistry, 7th Edition, 2007, Chapter Vitamins). These methods may be also applied for production of thiamin using an mRNA stabilizing element as described herein.

In one embodiment, the target fermentation product is pyridoxin (vitamin B6). As used herein, the term "pyridoxin" includes but is not limited to pyridoxin, pyridoxol, pyridoxal, pyridoxamine and precursors/derivatives thereof such as *e.g.* pyridoxal 5'-phosphate. The terms "pyridoxin" and "vitamin B6" are used interchangeably herein. The genes involved in biosynthesis of pyridoxin as well as methods for fermentative production of pyridoxin are known (see e.g. Mittenhuber, J. Mol. Microbiol. Biotechnol. 3(1), p. 1-20, 2001 or EP 950715 or Ullman's Encyclopedia of Industrial Chemistry, 7th Edition, 2007, Chapter Vitamins). These methods may be also applied for production of pyridoxin using an mRNA stabilizing element as described herein.

The target fermentation product may also be selected from enzymes, in particular selected from the group consisting of transferases [EC 2] and hydrolases [EC 3], preferably selected from glycosyltransferases [EC 2.4], glycosylases [EC 3.2] or peptidases [EC 3.4], more preferably selected from glycosidases [EC 3.2.1] or hexosyltranferases [EC 2.4.1], such as e.g. α-amylase, xylanase, β-glucanase, maltogenic amylase (glucan 1,4-α-maltohydrolase), neutral protease/proteinase or amylomaltase (4-α-glucanotransferase). The genes involved in biosynthesis of said enzymes as well as methods for fermentative production thereof are known (see *e.g.* WO 03/062409 or EP 585617 or WO 97/06181 or Sonenshein, Hoch, Losick (eds.): Bacillus subtilis and Other Gram-Positive Bacteria: Biochemistry, Physiology, and Molecular Genetics, ASM Press, 1993 or Ullman's Encyclopedia of Industrial Chemistry, 7th Edition, 2007, Chapter Enzymes). These methods may be also applied for production of enzymes using an mRNA stabilizing element as described herein.

Consequently, the invention relates to a polynucleotide selected from the group consisting of;
(a) polynucleotides comprising the nucleotide sequence according to SEQ ID NO:1 to 5;
(b) polynucleotides comprising a nucleotide sequence obtainable by nucleic acid amplification such as polymerase chain reaction, using genomic DNA from a microorganism as a template and a primer set according to SEQ ID NO:6 / SEQ ID NO:7, SEQ ID NO:8 / SEQ ID NO:9, SEQ ID NO:10 / SEQ ID NO:11, SEQ ID NO:12 / SEQ ID NO:13 and SEQ ID NO:14 / SEQ ID NO:15, respectively;
(c) polynucleotides comprising a nucleotide sequence which is a fragment or derivative of a polynucleotide according to (a) or (b), said fragment or derivative having the activity of an mRNA stabilizing element;
(d) polynucleotides the complementary strand of which hybridizes under stringent conditions to a polynucleotide as defined in any one of (a) to (c) and which have the activity of an mRNA stabilizing element; and
(e) polynucleotides which are at least 60%, such as 70, 85, 90 or 95% identical to a polynucleotide as defined in any one of (a) to (c) and which have the activity of an mRNA stabilizing element
   or
   the complementary strand of such a polynucleotide.

The nucleic acid sequence as disclosed herein and as isolated from *Bacillus subtilis* shown in SEQ ID NO: 1 to 5 was found to be particularly useful in stabilization of mRNA and thus helpful in the production of a target fermentation product as described above in a microorganism, in particular bacteria, such as Gram-positive and Gram-negative bacteria, such as for instance *Bacillus.*

The term "mRNA stabilizing element" as used herein refers to a DNA sequence which upon introduction in the 5'-untranslated region, *e.g.* downstream of the transcription start of the respective gene, is capable of providing an increase stability to the mRNA which is transcribed from the respective gene comprising said "mRNA stabilizing element". The mRNA stabilizing element preferably contains 1 or more stem loops.

The present invention provides stabilized mRNA sequences. The stabilized mRNA is transcribed from an endogenous gene containing a DNA sequence as defined above which is introduced downstream of the transcription start of the relevant gene(s). This gene may be involved in production of a desired target fermentation product as described above. The mRNA stabilizing element may be introduced directly downstream of the transcription start or 1 or more nucleotides downstream thereof. Introduction of said mRNA stabilizing element has the effect that the mRNA is no longer or less accessible to enzymatic degradation and thus results in higher production/yield/efficiency of the desired target fermentation product.

Any nucleic acid sequence capable of forming one or more stem loop(s) leading to increased stability of mRNA transcripts from one or more target gene(s) which are preferably involved in the production of a desired target fermentation product as defined above may be within the scope of the present invention. Stabilization of mRNA may also be possible via a strong ribosome binding site (RBS), as e.g. in the case of SEQ ID NO:5.

Thus, the present invention relates to mRNA stabilizing elements introduced at the 5'-end of an endogenous gene which upon transcription result in stabilized mRNA transcripts, i.e. which are capable of increasing the stability of mRNA transcripts.

A nucleic acid according to the invention may be obtained by nucleic acid amplification using *e.g.,* cDNA, mRNA or alternatively, genomic DNA as a template and appropriate oligonucleotide primers such as the nucleotide primers according to SEQ ID NO:6 / SEQ ID NO:7, SEQ ID NO:8 SEQ ID NO:9, SEQ ID NO:10 / SEQ ID NO:11, SEQ ID NO:12 / SEQ ID NO:13 and SEQ ID NO:14 / SEQ ID NO:15, respectively, according to standard PCR amplification techniques, such as for instance according to the process described in Example 1. The nucleic acid thus amplified may be cloned into an appropriate vector and characterized by DNA sequence analysis. Synthetic DNA may also be used as template for such PCR.

The template DNA may be derived from the same or a different host cell to be used for the production of the desired target fermentation product. Furthermore, the template for the reaction may be cDNA obtained by reverse transcription of mRNA prepared from strains known or suspected to comprise a polynucleotide according to the invention. The PCR product may be subcloned and sequenced to ensure that the amplified sequences represent the sequences of a new nucleic acid sequence as described herein, or a functional equivalent thereof. Furthermore, a nucleic acid sequence according to the present invention may be completely or partly synthesized using methods well-known in the art.

Accordingly, the invention relates to polynucleotides comprising a nucleotide sequence obtainable by nucleic acid amplification such as polymerase chain reaction, using DNA such as genomic DNA from a microorganism as a template and a primer set according to SEQ ID NO:6 / SEQ ID NO:7, SEQ ID NO:8 / SEQ ID NO:9, SEQ ID NO:10 / SEQ ID NO:11, SEQ ID NO:12 / SEQ ID NO:13 and SEQ ID NO:14 / SEQ ID NO:15, respectively.

The invention also relates to polynucleotides comprising a nucleotide sequence which is a fragment or derivative of a polynucleotide as described herein, said fragment or derivative having the activity of an mRNA stabilizing element. An example of such a fragment is shown in SEQ ID NO:16, 17, 18, 19 and/or 20.

The invention also relates to polynucleotides the complementary strand of which hybridizes under stringent conditions to a polynucleotide as defined herein and which have the activity of an mRNA stabilizing element.

The invention also relates to polynucleotides which are at least 60% identical to a polynucleotide as defined herein and which have the activity of an mRNA stabilizing element; and the invention also relates to polynucleotides being the complementary strand of a polynucleotide as defined herein above.

The invention also relates to primers, probes and fragments that may be used to amplify or detect a DNA according to the invention.

The mRNA stabilizing elements as defined above may be of any length but preferably consist of at least 15 nucleotides, more preferably at least 20, 30, 40, 50, 60, 70, 80, 90, 100 nucleotides, most preferably 39-87 nucleotides comprising preferably 1 or more stem loops, in particular 1 or 2 stem loops. The stem may consist of at least 4 base pairs, preferably at least 8, 10, 12,15 base pairs (with mismatch nucleotides/interior loops and/or bulge loops possibly being present) and the loops may consist of *e.g.* 3-30 unbounded nucleotides, preferably 4, 6, 8,10, 11,14, 15, 25 or more nucleotides. The length of the interior loops is preferably 2, 4, 6, 8,10, 12 unbounded nucleotides. One or more bulge loop(s) may be present, consisting of *e.g.* 1, 2 or even 6 unbounded nucleotides. The calculated thermodynamic stability (ΔG) of the stem loop may be calculated according to algorithms developed by Zuker (2003, Nucleic Acids Res. 31:3406-3415). In one embodiment, the calculated thermodynamic stability is -2.8 kcal/mol or lower, preferably - 3, -4, -5, -6, -7, -8, -9, -10, -11, -12, -15, -20 kcal/mol or lower.

The invention also relates to processes for producing microorganisms genetically engineered with a polynucleotide as defined above which are capable of producing an mRNA transcript with increased and/or improved stability.

The invention also relates to microorganisms, which are genetically engineered with the nucleic acid sequences as defined above as well as to processes for producing said genetically engineered cells.

The invention also relates to genetically engineered microorganisms wherein the yield and/or efficiency of production of a target fermentation product as defined above is improved and/or enhanced and to microorganisms wherein the stability of an mRNA transcripted from a gene involved in the biosynthetic pathway of a target fermentation product as defined above is increased and/or improved so that the yield of the target fermentation product which is produced from a carbon source is increased.

Furthermore, the invention is related to the use of nucleic acid sequences, e.g. mRNA stabilizing elements as defined above or to the use of a genetically engineered microorganisms for the production of a target fermentation product as defined above.

The skilled person will know how to generate such genetically engineered cells, in particular microorganisms, *i.e.* how to introduce a DNA sequence of the present invention as defined above leading to stabilized transcripts. Introduction of a DNA sequence as used herein may be for instance addition or insertion of a DNA sequence by transformation, conjugation or transduction into the chromosome of a host cell. Said addition or insertion may occur by DNA recombination that may or may not also result in a removal or deletion of chromosomal DNA nucleotides. Methods by which introduction of DNA sequences into a host cell, e.g. microorganisms, are achieved, especially by site-specific introduction, are well-known in the art and described in e.g. Sambrook et al., 1989, Molecular Cloning, A Laboratory Manual, Cold Spring Harbor Press, N. Y.; and Ausubel et al. (eds.), 1995, Current Protocols in Molecular Biology, (John Wiley & Sons, N.Y.). The DNA sequence capable of forming one or more stem loops may be introduced downstream of the transcription start, in particular at least 1, 2, 3, 5, 6, 7 or more, e.g. 8, 9, 10, 11, 12, 13 nucleotides downstream of the transcription start site of the relevant gene(s) involved in biosynthesis of a desired target fermentation product. The number and kind of nucleotide changes are limited by the fact that no sequence is formed representing an RNase E-specific nuclease cleaving site.

The stability of mRNA transcripts may be for instance measured by determination of the mRNA half-life using Northern blot and/or real-time PCR stabilization as described *e.g.* by Allenby et al., Microbiology, 150, p.2619-2628 (2004) or Sharp and Bechhofer, Mol. Microbiol. 57, 484-495 (2005). As used herein, the mRNA stability is increased (or mRNA degradation is reduced/blocked) if the half-life of said mRNA is increased by at least 1%, 2%, 5%, 10%, 25%, 50%, 75%, 100%, 200% or even more than 500%, compared to mRNA half-life transcribed from a wild-type gene, *i.e.* not containing an mRNA stabilizing element as of the present invention.

The invention may be performed in/with any microorganism carrying the gene(s) involved in the biosynthesis of the above-defined target fermentation products. Suitable microorganisms may be selected from e.g. bacteria, fungi (including yeast) and algae. The term bacteria includes both Gram-negative and Gram-positive microorganisms. Suitable bacteria may be selected from *e.g. Escherichia, Gluconobacter, Rhodobacter, Pseudomonas, Paracoccus, Bacillus, Brevibacterium, Corynebacterium, Rhizobium (Sinorhizobium), Flavobacterium, Klebsiella, Enterobacter, Lactobacillus, Lactococcus* or *Streptomyces.* Preferably, the microorganism or host cell is selected from the group consisting of *B. subtilis, B. amyloliquefaciens, B. licheniformis, B. puntis, B. halodurans, B. pumilus, G. oxydans, Rhodobacter sphaeroides and Pseudomonas zeaxanthinifaciens, Paracoccus denitrificans, E. coli, C. glutamicum, Streptomyces lividans, Sinorhizobium melioti* and *Rhizobium radiobacter.* Examples of yeasts are *Saccharomyces,* particularly *S. cerevisiae, Pichia* or *Candida.* Examples of preferred other fungi are *Aspergillus* and *Pencillium,* in particular *A. niger and P. chrysogenum.*

Microorganisms which can be used for the present invention may be publicly available from different sources, e.g., Deutsche Sammlung von Mikroorganismen und Zellkulturen (IaSMZ), Mascheroder Weg 1B, D-38124 Braunschweig, Germany, American Type Culture Collection (ATCC), P.O. Box 1549, Manassas, VA 20108 USA, Agricultural Research Culture Collection (NRRL), Peoria, IL, USA, Culture Collection Division, NITE Biological Resource Center, 2-5-8, Kazusakamatari, Kisarazu-shi, Chiba, 292-0818, Japan (formerly: Institute for Fermentation, Osaka (IFO),17-85, Juso-honmachi 2-chome,Yodogawa-ku, Osaka 532-8686, Japan) or from the *Bacillus* Genetic Stock Center (BGSC), The Ohio State University, Columbus, Ohio 43210 USA. An example of a preferred bacteria is for instance *B. subtilis* 1A747 obtainable from BGSC, which is a derivative of *B. subtilis* 168.

In connection with the above process using a microorganism it is understood that the above-mentioned microorganisms also include synonyms or basonyms of such species having the same physiological properties, as defined by the International Code of Nomenclature of Prokaryotes. The nomenclature of the microorganisms as used herein is the one officially accepted (at the filing date of the priority application) by the International Committee on Systematics of Prokaryotes and the Bacteriology and Applied Microbiology Division of the International Union of Microbiological Societies, and published by its official publication vehicle International Journal of Systematic and Evolutionary Microbiology (IJSEM).

Examples of preferred strains for the production of *e.g.* riboflavin are selected from *B. subtilis, B. licheniformis, B. amyloliquefaciens, B. ammoniagenes, E. coli* and *C. glutamicum.* A more preferred strain is *B. subtilis* RB50::[pRF69]ₙ containing multiple (n) copies (for example about 5 to about 20 copies) of pRF69 encoding a *rib* operon modified with the strong phage SPO1 promoter (*P*₁₅) to enhance transcription of the *rib* genes (see e.g. EP 405370 and Perkins et al., J. Ind. Microbiol. Biotechnol., 22:8-18, 1999 for construction of the strain and culture conditions to result in riboflavin production). *B. subtilis* RB50 and plasmid pRF69 may be available from NRRL (accession number B 18502) and from ATCC (accession number ATCC 68338), respectively.

Examples of preferred strains for production of *e.g.* biotin are selected from *B. subtilis, B. sphaericus* and *E. coli,* more preferably strains available from ATCC, such as *e.g. B. subtilis* PA3, HB43, HB3, BI544, BI535, BI421, BI304, BI282, and BI274 (accession numbers ATCC 55567 to ATCC 55575) as disclosed in EP 635572.

Examples of preferred strains for production of e.g. pantothenic acid are selected from *B*. *subtilis,* such as *B. subtilis* 168, *B. licheniformis, B. amyloliquefaciens, B. puntis, B. halodurans* and *C*. *glutamicum.*

Examples of preferred strains for production of e.g. thiamin are selected from *B. subtilis* and *E*. *coli,* more preferably strains as deposited under the terms of the Budapest treaty with ATCC and DSMZ, respectively, such as *e.g. B. subtilis* TH95, *B. subtilis* TH101, *B. subtilis* TH115, *B*. *subtilis* TH116 (accession numbers ATCC PTA-5221 to ATCC PTA-5224) or *B. subtilis* TH404 and *B. subtilis* TH405 (accession numbers DSM 16333 and 16334).

Examples of preferred strains for production of *e.g.* pyridoxin are selected from *B. subtilis, E. coli, S. melioti, P. putida, L. brevis, F. indolgenes, C. ammoniagenes, C. glutamicum, P. guilliermondii, S. cerevisiae, C. tropicalis, E. cloacae, P. maltophila,* more preferably strains available from NITE Biological Resource Center such as *e.g. Sinorhizobium melioti* (accession number IFO 14782 or DSM 10226), *Flavobacterium indologenes* (accession number IFO 14944), *Lactobacillus brevis* (accession number IFO 13110), *Bacillus subtilis* (accession number IFO 3007), *Klebsiella planticola* (accession number IFO 3317), *Escherichia coli* (accession number IFO 13168), *Pseudomonas putida* (accession number IFO 3738), *Pseudomonas maltophila* (accession number IFO 12692), *Enterobacter cloacae* (accession number IFO 3320), *Corynebacterium ammoniagenes* (accession number IFO 12612), *Corynebacterium glutamicum* (accession number IFO 12168), *Brevibacterium acetylicum* (accession number IFO 12146), *Pichia guilliermondii* (accession number IFO 10106), *Saccharomyces cerevisiae* (accession numbers IFO 0304 and IFO 0306) and *Candida tropicalis* (accession numbers IFO 0199 and IFO 0587).

Examples of preferred strains for production of enzymes such as e.g. transferases [EC 2] or hydrolases [EC 3] are selected from *B. licheniformis, B. amyloliquefaciens,* and *B. subtilis.*

The invention also relates to processes for the expression of endogenous gene(s) involved in the biosynthesis of a target fermentation product as *e.g.* defined above within a microorganism, wherein the mRNA transcribed from said gene(s) has increased stability. This increased stability is achieved through introduction of an mRNA stabilizing element as defined above into the microorganism. As a result, the mRNA thus generated is more stable than the mRNA generated from the corresponding wild-type gene.

The present invention is also related to the production of microorganisms capable of producing the target fermentation product, wherein the productivity and/or yield of said target fermentation product is increased compared to the wild-type organism. This increase is obtained via altering said microorganism by introduction of a polynucleotide as of the present invention so that the microorganism produces a more stable mRNA.

Thus, the present invention is directed to a process for the production of a target fermentation product with a microorganism wherein said microorganism is incubated in a aqueous medium under conditions that allow the production of said target fermentation product from a carbon source and wherein optionally the target fermentation product is isolated as the fermentation product, wherein said microorganism is genetically altered by introduction of a polynucleotide as defined above that it leads to an improved yield and/or efficiency of production of the target fermentation product produced by said microorganism.

Furthermore, the present invention discloses a process for the production of mRNA with increased stability transcribed from a gene involved in the biosynthetic pathway of a target fermentation product in a microorganism, said microorganism comprising a polynucleotide as defined above introduced downstream of the transcription start of the respective gene(s).

As a further embodiment, the present invention is related to a process for the production of a microorganism capable of producing a target fermentation product, said process comprising the step of altering said microorganism by introduction of a polynucleotide as defined above so that the microorganism produces a stabilized mRNA leading to an improved yield and/or efficiency of production of the target fermentation product produced by said microorganism as well as to a process for the production of a microorganism wherein the stability of mRNA transcribed from an endogenous gene is increased, comprising the step of altering said microorganism by introduction of a polynucleotide as defined above downstream of the transcription start of the respective endogenous gene.

All these processes may comprise the step of altering a microorganism, wherein "altering" as used herein encompasses the process for "genetically altering" in such a way that (i) the yield and/or productivity of the fermentation product and/or (ii) the stability of an mRNA transcript can be improved compared to the wild-type organism. This is achieved by introducing a nucleic acid sequence as defined above into the respective organism.

The term "genetically engineered" or "genetically altered" means the scientific alteration of the structure of genetic material in a living organism. It involves the production and use of recombinant DNA. More in particular it is used to delineate the genetically engineered or modified organism from the naturally occurring organism, Genetic engineering may be done by a number of techniques known in the art, such as *e.g.* gene replacement, gene amplification, gene disruption, addition, insertion, deletion, transfection, transformation using plasmids, viruses, or other vectors. A genetically modified organism, e.g. genetically modified microorganism, is also often referred to as a recombinant organism, e.g. recombinant microorganism.

The nucleic acid sequence as defined in SEQ ID NO:1 to 5 was determined by sequencing a genomic clone obtained from *B, subtilis.*

The invention also relates to fragments and/or derivatives of a nucleotide sequence according to SEQ ID NO:1, 2, 3, 4 and/or 5 having the activity of an mRNA stabilizing element, *i.e.* leading to transcripts which are more stable than a wild-type mRNA. Said fragments/derivatives may consist of 1 or more, in particular 1 or 2, stem-loop(s). An example of such a fragment is shown in SEQ ID NO:16, 17, 18, 19 or 20..

The nucleic acids of the present invention are preferably provided in an isolated form, and preferably purified to homogeneity,

The term "isolated" means that the material is removed from its original environment (e.g., the natural environment if it is naturally occurring). For example, a naturally-occurring polynucleotide present in a living microorganism is not isolated, but the same polynucleotide, separated from some or all of the coexisting materials in the natural system, is isolated. Such polynucleotides could be part of a vector and/or such polynucleotides could be part of a composition and still be isolated in that such vector or composition is not part of its natural environment.

As used herein, the term "gene" refer to nucleic acid molecules which may be isolated from chromosomal DNA, which include an open reading frame encoding a protein, *e.g.* proteins involved in the synthesis of the desired target fermentation product, such as for instance enzymes from the *B. subtilis* riboflavin biosynthetic pathway.

A gene may include coding sequences, non-coding sequences such as for instance untranslated sequences located at the 3'- and 5'-ends of the coding region of a gene, such as for instance promoter regions, regulator regions and terminator regions important for the appropriate expression and stabilization of the polypeptide derived thereof.

As used herein, the terms "polynucleotide" or "nucleic acid molecule" are intended to include DNA molecules (e.g., cDNA or genomic DNA) and RNA molecules (e.g., mRNA) and analogs of the DNA or RNA generated using nucleotide analogs. The nucleic acid molecule may be single-stranded or double-stranded, but preferably is double-stranded DNA. The nucleic acid may be synthesized using oligonucleotide analogs or derivatives (e.g., inosine or phosphorothioate nucleotides). Such oligonucleotides may be used, for example, to prepare nucleic acids that have altered base-pairing abilities or increased resistance to nucleases.

Unless otherwise indicated, all nucleotide sequences determined by sequencing a DNA molecule herein were determined using an automated DNA sequencer. Therefore, as is known in the art for any DNA sequence determined by this automated approach, any nucleotide sequence determined herein may contain some errors. Nucleotide sequences determined by automation are typically at least about 90% identical, more typically at least about 95% to at least about 99.9% identical to the actual nucleotide sequence of the sequenced DNA molecule. The actual sequence may be more precisely determined by other approaches including manual DNA sequencing methods well known in the art.

The person skilled in the art is capable of identifying such erroneously identified bases and knows how to correct for such errors.

A nucleic acid molecule according to the invention may comprise only a portion or a fragment of the nucleic acid sequence provided by the present invention, such as for instance the sequence shown in SEQ ID NO:1 to 5, for example a fragment which may be used as a probe or primer such as for instance SEQ ID NO:6, 7, 8, 9, 10, 11, 12, 13, 14 or 15 or a fragment consisting of the stem and one or more loops of the mRNA stabilizing element, such as e.g. shown in SEQ ID NO:16, 17, 18, 19 or 20. The probe/primer typically comprises substantially purified oligonucleotides which typically comprises a region of nucleotide sequence that hybridizes preferably under highly stringent conditions to at least about 12 or 15, preferably about 18 or 20, more preferably about 22 or 25, even more preferably about 30, 35, 40, 45, 50, 55, 60, 65, or 75 or more consecutive nucleotides of a nucleotide sequence shown in SEQ ID NO:1, 2, 3, 4, 5 or a fragment or derivative thereof.

A nucleic acid molecule encompassing all or a portion of the nucleic acid sequence of SEQ ID NO:1 may be also isolated by the polymerase chain reaction (PCR) using synthetic oligonucleotide primers designed based upon the sequence information contained herein.

The invention also relates to an isolated polynucleotide hybridizable under stringent conditions, preferably under highly stringent conditions, to a polynucleotide as of the present invention, such as for instance a polynucleotide shown in SEQ ID NO:1 to 5. Advantageously, such polynucleotide may be obtained from a microorganism capable of producing the above-defined target fermentation product, in particular *Bacillus subtilis.*

As used herein, the term "hybridizing" is intended to describe conditions for hybridization and washing under which nucleotide sequences at least about 50%, at least about 60%, at least about 70%, more preferably at least about 80%, even more preferably at least about 85% to 90%, most preferably at least 95% homologous to each other typically remain hybridized to each other.

In one embodiment, a nucleic acid of the invention is at least 40%, 45%, 50%, 55%, 60%, 65%, 70%, 75%, 80%, 85%, 90%, 91 %, 92%, 93%, 94%, 95%, 96%, 97%, 98%, 99%, or more homologous to a nucleic acid sequence shown in SEQ ID NO:1 or the complement thereof.

A preferred, non-limiting example of stringent hybridization conditions are hybridization in 6x sodium chloride/sodium citrate (SSC) at about 45°C, followed by one or more washes in 1x SSC, 0.1% SDS at 50°C, preferably at 55°C, more preferably at 60°C and even more preferably at 65°C.

Highly stringent conditions include incubations at 42°C for a period of several days, such as 2-4 days, using a labeled DNA probe, such as a digoxigenin (DIG)-labeled DNA probe, followed by one or more washes in 2x SSC, 0.1 % SDS at room temperature and one or more washes in 0.5x SSC, 0.1% SDS or 0.1x SSC, 0.1% SDS at 65-68°C. In particular, highly stringent conditions include, for example, 2 h to 4 days incubation at 42°C using a DIG-labeled DNA probe (prepared by e.g. using a DIG labeling system; Roche Diagnostics GmbH, 68298 Mannheim, Germany) in a solution such as DigEasyHyb solution (Roche Diagnostics GmbH) with or without 100 µg/ml salmon sperm DNA, or a solution comprising 50% formamide, 5x SSC (150 mM NaCl, 15 mM trisodium citrate), 0.02% sodium dodecyl sulfate, 0.1% N-lauroylsarcosine, and 2% blocking reagent (Roche Diagnostics GmbH), followed by washing the filters twice for 5 to 15 minutes in 2x SSC and 0.1% SDS at room temperature and then washing twice for 15-30 minutes in 0.5x SSC and 0.1% SDS or 0.1x SSC and 0.1% SDS at 65-68°C.

The skilled artisan will know which conditions to apply for stringent and highly stringent hybridization conditions. Additional guidance regarding such conditions is readily available in the art, for example, in Sambrook et al., 1989, Molecular Cloning, A Laboratory Manual, Cold Spring Harbor Press, N. Y.; and Ausubel et al. (eds.), 1995, Current Protocols in Molecular Biology, (John Wiley & Sons, N.Y.).

A nucleic acid molecule of the present invention, such as for instance a nucleic acid molecule shown in SEQ ID NO:1 to 5 or a fragment or derivative thereof, may be isolated using standard molecular biology techniques and the sequence information provided herein. For example, using all or portion of the nucleic acid sequence shown in SEQ ID NO: 1 to 5 as a hybridization probe, nucleic acid molecules according to the invention may be isolated using standard hybridization and cloning techniques (*e.g.,* as described in Sambrook et al., supra).

Furthermore, oligonucleotides corresponding to or hybridizable to nucleotide sequences according to the invention may be prepared by standard synthetic techniques, *e.g.,* using an automated DNA synthesizer.

The terms "homology" or "percent identity" are used interchangeably herein. For the purpose of this invention, it is defined here that in order to determine the percent identity of two nucleic acid sequences, the sequences are aligned for optimal comparison purposes (e.g., gaps may be introduced in the sequence of a first nucleic acid sequence for optimal alignment with a second nucleic acid sequence). The nucleotides at corresponding positions are then compared. When a position in the first sequence is occupied by the same nucleotide as the corresponding position in the second sequence, then the molecules are identical at that position. The percent identity between the two sequences is a function of the number of identical positions shared by the sequences [*i.e.,* % identity = number of identical positions/total number of positions *(i.e.,* overlapping positions) x 100]. Preferably, the two sequences are the same length.

The skilled person will be aware of the fact that several different computer programs are available to determine the homology between two sequences. For instance, a comparison of sequences and determination of percent identity between two sequences may be accomplished using a mathematical algorithm. In a preferred embodiment, the percent identity between two amino acid sequences is determined using the Needleman and Wunsch (J. Mol. Biol. 48, 444-453, 1970) algorithm which has been incorporated into the GAP program in the GCG software package (available at http://www.accelrys.com), using either a Blossom 62 matrix or a PAM250 matrix, and a gap weight of 16, 14,12, 10, 8, 6 or 4 and a length weight of 1, 2, 3, 4, 5 or 6. The skilled person will appreciate that all these different parameters will yield slightly different results but that the overall percentage identity of two sequences is not significantly altered when using different algorithms.

In yet another embodiment, the percent identity between two nucleotide sequences is determined using the GAP program in the GCG software package (available at http://www,accelrys.com), using a NWSgapdna.CMP matrix and a gap weight of 40, 50, 60, 70 or 80 and a length weight of 1, 2, 3, 4, 5 or 6. In another embodiment, the percent identity between two nucleotide sequences is determined using the algorithm of E. Meyers and W. Miller (CABIOS, 4: 11-17, 1989) which has been incorporated into the ALIGN program (version 2.0) (available at http://vega.igh.cnrs.fr/bin/align-guess,cgi) using a PAM120 weight residue table, a gap length penalty of 12 and a gap penalty of 4.

The nucleic acid sequences, *i.e.* mRNA stabilizing elements, as of the present invention may be operatively linked to an appropriate promoter, which may be either a constitutive or inducible promoter. The promoter will be either the natural one or a promoter which is originally not naturally linked to the respective gene(s) involved in biosynthesis of a target fermentation product. The skilled person will know how to select suitable promoters. The expression constructs may contain sites for transcription initiation, termination, and, in the transcribed region, a ribosome binding site for translation. The coding portion of the mature transcripts expressed by the constructs may preferably include an initiation codon at the beginning and a termination codon appropriately positioned at the end of the polypeptide to be translated.

Promoters suitable for the expression of respective gene(s) involved in biosynthesis of a desired target fermentation product useful for the present invention can be found in the literature, see *e.g.* EP 405370, EP 635572, WO 01/21772, WO 04/106557, EP 950715 or WO 03/062409 for promoters used in the fermentative production of riboflavin, biotin, pantothenic acid, pyridoxin and enzymes selected from transferases or hydrolases, respectively.

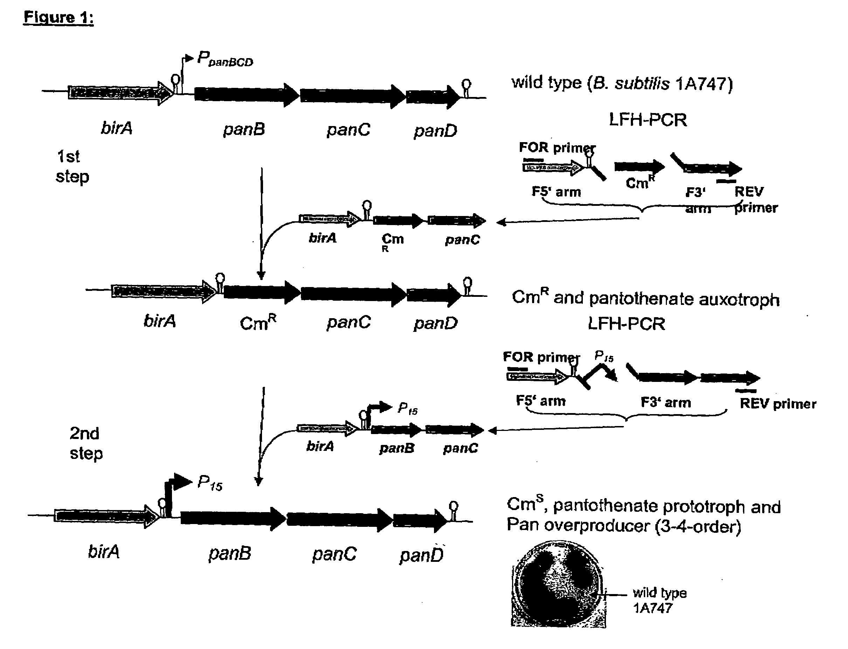

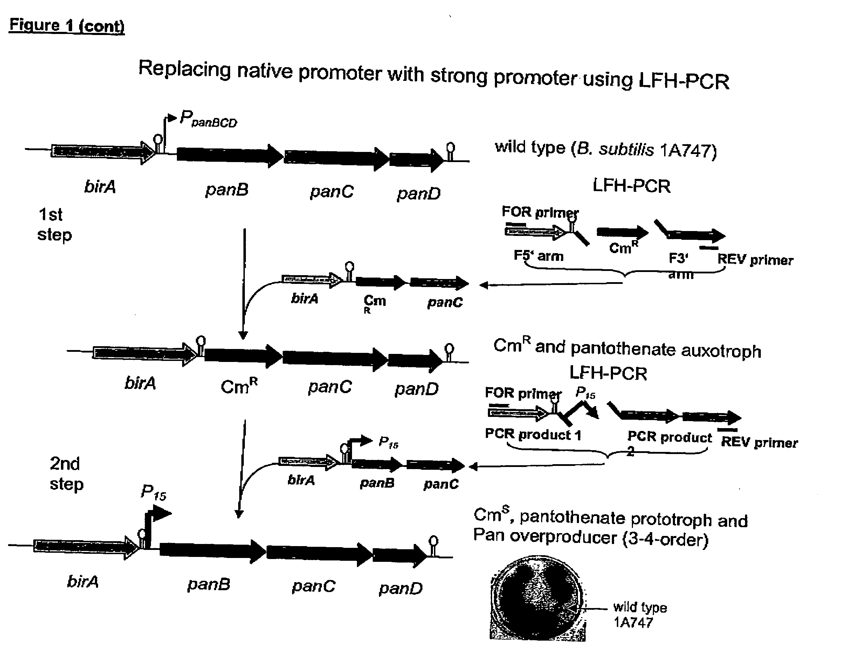

A useful method for constructing a microorganism as of the present invention, *i.e.* introducing a stabilizing mRNA element downstream of the transcription start of a gene involved in production of a desired target fermentation product is shown in Figure 1, wherein the gene(s) downstream of the promoter maybe any gene involved in biosynthesis of the desired target fermentation product, such as, *e.g*. panB, bioA, ribD, amyQ, wherein introduction of a herein disclosed mRNA stabilizing element leads to a more stable mRNA transcripts from the respective gene(s) which furthermore leads to increased yield and/or productivity of the respective target fermentation product. The sequences of said gene(s) involved in biosynthesis of the desired products are publicly available as known by the skilled person. Selection of recombinant microorganisms can be performed via introduction of an antibiotic resistance gene, such as for instance chloramphenicol, neomycin, streptomycin, spectinomycin or the like. Optionally, the natural promoter may furthermore replaced by a stronger such as *e.g.* the P₁₅ constitutive strong promoter.

As mentioned above, the nucleic acid sequences as of the present invention may be utilized in the genetic engineering of a suitable host cell to make it better and more efficient in the fermentation, for example in production of a target fermentation product as defined herein.

According to the invention a genetically cngincered/recombinantly produced host cell (also referred to as recombinant cell or transformed cell) carrying such a mRNA stabilizing element as of the present invention such that the yield, production and/or efficiency of production of a target fermentation product, in particular vitamins or enzymes as defined herein, is improved. The host cell may be selected from a microorganism capable of producing said target fermentation product such as for instance vitamin B2, pantothenic acid, transferases or hydrolases from a given carbon source, in particular *Bacillus,* preferably *B. subtilis.*

A "transformed cell" or "recombinant cell" is a cell into which (or into an ancestor of which) has been introduced, by means of recombinant DNA techniques, a nucleic acid according to the invention leading to increased and/or enhanced stability of mRNA transcribed from the respective gene(s), Suitable host cells include cells of microorganisms capable of producing a given fermentation product, *e.g.,* converting a given carbon source into a target product as defined above. Useful strains for performing said fermentation process are listed above and known in the art.

The present invention provides for a process for the production of a target fermentation product such as, *e.g.* riboflavin, biotin, pantothenic acid, folic acid, thiamin, pyridoxin, xylanase, amylase, protease, glucanase, amylomaltase or maltogenic amylase, wherein the production/yield of said target fermentation products using a recombinant organism is increased compared to a non-modified organism. As used herein, "improved yield of target fermentation product" means an increase of at least 5%, 10%, 25%, 30%, 40%, 50%, 75%, 100%, 200% or even more than 500%, compared to a wild-type microorganism, *i.e.* a non-modified microorganism.

Several substrates may be used as a carbon source in a process of the present invention, i. e. a process for production of a target fermentation product as mentioned above. Particularly suited carbon sources may be selected from compounds consisting of 3, 5 or 6 carbon atoms, such as e.g. D-glucose, glycerol, thick juice, dextrose, starch, sucrose or ribose. Preferably, the carbon source is D-glucose. The term "carbon source", "substrate" and "production substrate" in connection with the above process using a microorganism is used interchangeably herein.

A medium as used herein for the above process using a microorganism may be any suitable medium for the production of the desired target fermentation product. Typically, the medium is an aqueous medium comprising for instance salts, substrate(s), and a certain pH. The medium in which the substrate is converted into the desired product is also referred to as the production medium.

"Fermentation" or "production" or "fermentation process" as used herein may be the use of growing cells using media, conditions and procedures known to the skilled person, or the use of non-growing so-called resting cells, after they have been cultivated by using media, conditions and procedures known to the skilled person, under appropriate conditions for the conversion of suitable substrates into desired products such as e.g. vitamins or enzymes.

In one embodiment a microorganism is capable of the conversion of a certain substrate into the specified product by means of one or more biological conversion steps, without the need of any additional chemical conversion step. Said microorganism is cultured under conditions which allow such conversion from the substrate as defined above.

The produced target fermentation product may be recovered from the cells by any suitable means. Recovering means for instance that the produced fermentation product may be separated from the production medium. Optionally, the thus produced target fermentation product may be further processed.

In connection with the above process using a microorganism, in one aspect, the growing step can be performed in an aqueous medium, *i.e.* the growth medium, supplemented with appropriate nutrients for growth normally under aerobic conditions. The cultivation may be conducted, for instance, in batch, fed-batch, semi-continuous or continuous mode, wherein fed-batch or semi-continuous mode is preferred. The cultivation period may vary depending on for instance the host, target fermentation product, pH, temperature and nutrient medium to be used, and may be for instance about 10 h to about 10 days, preferably about 4 to about 7 days, more preferably about 2 to about 6 days, depending on the microorganism. If the microorganism is selected from bacteria, the cultivation may be conducted for instance at a pH of about 7.0, preferably in the range of about 6 to about 8, more preferably about 6.5 to 7.5. A suitable temperature range for carrying out the cultivation using bacteria may be for instance from about 13°C to about 70°C, preferably from about 35°C to about 39°C, more preferably from about 30°C to about 39°C, and most preferably from about 36°C to about 39°C. The culture medium for growth usually may contain such nutrients as assimilable carbon sources, e.g., D-glucose, glycerol, thick juice, dextrose, starch, sucrose or ribose; and digestible nitrogen sources such as organic substances, e.g., peptone, yeast extract and amino acids. The media may be with or without urea and/or corn steep liquor and/or baker's yeast, Various inorganic substances may also be used as nitrogen sources, e.g., nitrates and ammonium salts. Furthermore, the growth medium, usually may contain inorganic salts, *e.g.,* magnesium sulfate, manganese sulfate, potassium phosphate, and calcium carbonate. Cells obtained using the procedures described above can then be further incubated at essentially the same modes, temperature and pH conditions as described above, in the presence of substrates such as described above in such a way that they convert these substrates into the desired target fermentation product. Incubation can be done in a nitrogen-rich medium, containing, for example, organic nitrogen sources, e.g., peptone, yeast extract, baker's yeast, urea, amino acids, and corn steep liquor, or inorganic nitrogen sources, e.g., nitrates and ammonium salts, in which case cells will be able to further grow while producing the desired target fermentation product. Alternatively, incubation can be done in a nitrogen-poor medium, in which case cells will not grow substantially, and will be in a resting cell mode, or biotransformation mode. In all cases, the incubation medium may also contain inorganic salts, e.g., magnesium sulfate, manganese sulfate, potassium phosphate, and calcium chloride. An example of a suitable medium for production of a desired target fermentation product as described herein is described in WO 04/113510 (VF-medium), which is particularly useful with regards to *Bacillus.*

Analytical methods for determining the yield/productivity of a given target fermentation product are known in the art. Such methods may include, but are not limited to HPLC or use of indicator strains: for riboflavin, see e.g. Bretzel et al., J. Ind. Microbiol. Biotechnol. 22, 19-26, 1999; for biotin, see e.g. EP 635572 or Tanaka et al., J. Micro. Methods 6, 237-247,1987; for pantothenic acid, see *e.g.* WO 04/113510; for folic acid, see *e.g.* Ullman's Encyclopedia of Industrial Chemistry, 7th Edition, 2007, Chapter Vitamins; for thiamin, see *e.g.* WO 04/106557; for pyridoxin, see *e.g.* Tazoe et al., Biosci. Biotechnol. Biochem. 63 (8), 1378-1382,1999.

Methods for fermentation, isolation/purification and analytical methods with regards to target fermentation products selected from enzymes and which may be applicable for the present invention are disclosed in e.g. Rothstein et al., J. Bacteriol. 168 (2), 839-842, 1986 or Skolpap et al., Biotechnol. Bioeng. 86, 706-717, 2004 or Malhotra et al., Letters in Appl. Microbiol. 31, 378-384, 2000 (α-amylase from B. licheniformis, *B. subtilis* or B. thermooleovorans); Tang et al., Bioresource Technology 93, 175-181, 2004 (β-glucanase from *B. subtilis);* Heineken and O'Connor, J. Gen. Microbiol. 73, 35-44, 1972 or Calik et al., Biotechnol. Bioeng. 69, 301-311, 2000 (neutral protease from *B. subtilis* and B. licheniformis, respectively); Schneider et al., Can. J. Microbiol. 46, 784-789, 2000 or Mamo and Gessesse, Appl. Biochem. Biotechnol. 87, 95-101, 2000 or Sindhu et al., Current Microbiol. 53, 167-172, 2006 (xylanase from e.g. B. megaterium).

The following examples are illustrative only and are not intended to limit the scope of the invention in any way.

Figure 1: the 3 different steps for constructing a genetically altered microorganism carrying an mRNA stabilizing element downstream of a strong P₁₅ promoter via LFH-PCR are exemplified for the pantothenate (pan) operon comprising the genes panB, panC, panD. For selection of recombinant microorganisms, the chloramphenicol (Cm) antibiotic resistance gene is used. For more explanation see the examples.

### Examples

The following media as referred to in the examples are described in WO 04/106557: Tryptose Blood Agar Broth (TBAB) medium, Veal infusion-Yeast Extract broth (VY) medium, 10X Spizizen salts and Minimal Medium (MM).

100X Trace elements solution A: 12.5 g MgCl₂·6H₂O; 0.55 g CaCl₂; 1.35 g FeCl₂·6H₂O; 0.1 g MnCl₂·4H₂O; 0.17 g/l ZnCl₂; 0.043 g CuCl₂·2H₂O; 0.06 g CoCl₂·6H₂O; 0.06 g Na₂MoO₄·2H₂O; ad 11H₂O, autoclaved.

5X Minimal Salt Solution: 0.057 M K₂SO₄; 0.31 M K₂HPO₄·5H₂O; 0.22 M KH₂PO4; 0.017 M Na-citrate-7H₂O; 0.004 M MgSO₄, H₂O, pH 7.0, autoclaved.

100X Trace elements solution B: 0.55g CaCl₂; 0.17g ZnCl₂; 0.043g CuCl₂·2H₂O; 0.06 CoCl₂·6H₂O; 0.06g Na₂MoO₄·2H₂O; ad 11H₂O, autoclaved.

The sequences used with the respective ID numbers are shown in Table 1:

| ID NO: | Nucleotide sequence (5' to 3') |
|---|---|
| 1 | ACAGAATAGTCTMAAGTAAGTCTACTCTGAATTTTTTTA |
| 2 | ATTTTATCGAAGGGCAGCACCTGTCCTTCTCCTTACACTTTGAGGGAGG TGAACACA |
| 3 | AAGGATCTTCATCCTTAACATATTTTT |
| 4 | GGAGCCGCTGAGCTACCACAGATTGTGAAAGGAGAGGTTAAC |
| 5 | AAAGGAGGAATTCAAAATG |
| 6 | |
| 7 | |
| 8 | |
| 9 | |
| 10 | |
| 11 | |
| 12 | |
| 13 | |
| 14 | |
| 15 | |
| 16 | CAGAATAGTCTTTTAAGTAAGTCTACTCTG |
| 17 | TTTATCGAAGGGCAGCACCTGTCCTTCTCCTTACACTTTGAGGGAGGTG AA |
| 18 | AAGGATCTTCATCCTT |
| 19 | GGAGCCGCTGAGCTACC |
| 20 | AAAGGAGG |
| 21 | GATCCGGCGCGCCGGCCGGCCGTTTAAACGCGGCCGCGCATGCCCTG CAGGA |
| 22 | AGCTTCCTGCAGGGCATGCGCGGCCGCGTTTAAACGGCCGGCCGGCG CGCCG |
| 23 | CTCAAATTGTAAGTTTATTTCATTGCGTACTTTAAAAAGGATCGCTATAAT AACCAATAAGGACACAGAATAGTCTTTTAAGTAAGTCTACTCTGAATTTT TTTAGGTGGTGAACTACTGTGGAAGTTACTGACGTAAGATTACGG |
| 24 | CTCAAATTGTAAGTTTATTTCATTGCGTACTTTAAAAAGGATCGCTATAAT AACCAATAAGGACAATTTTATCGAAGGGCAGCACCTGTCCTTCTCCTTA CACTTTGAGGGAGGTGAACACA |
| 25 | CTCAAATTGTAAGTTTATTTCATTGCGTACTTTAAAAAGGATCGCTATAAT AACCAATAAGGACAAAGGATCTTCATCCTTAACATATTTTTGGTGGTGAA CTACTGTGGAAGTTACTGACGTAAGATTACGG |
| 26 | GATCTTTAGTTTGTTCTACCATGTTTTTATCACCTAAAAGTTTACCACTAA TTTTTGTTTATTATATCATAAACGGTGAAGCAATAATGGAGGAATGGTTG ACTTCAAAACAAATAAATTATATAATGACCTTTGTGTGAAAT |
| 27 | GATCTTTAGTTTGTTCTACCATGTTTTTATCACCTAAAAGTTTACCACTAA TTTTTGTTTATTATATCATAAACGGTGAAGCAATAATGGAGGAATGGTTG ACTTCAAAACAAATAAATTATATAATGACCTTTGTGTGAAATCAGAATAG TCTTTTAAGTAAGTCTACTCTGAATTTTTTTAGGTGGTGAACTACTGTGG AAGTTACTGACGTAAGATTACGG |
| 28 | GATCTTTAGTTTGTTCTACCATGTTTTTATCACCTAAAAGTTTACCACTAA TTTTTGTTTATTATATCATAAACGGTGAAGCAATAATGGAGGAATGGTTG ACTTCAAAACAAATAAATTATATAATGACCTTTGTGTGAAATATTTATCG AAGGGCAGCACCTGTCCTTCTCCTTACACTTTGAGGGAGGTGAACACA |
| 29 | GATCTTTAGTTTGTTCTACCATGTTTTTATCACCTAAAAGTTTACCACTAA TTTTTGTTTATTATATCATAAACGGTGAAGCAATAATGGAGGAATGGTTG ACTTCAAAACAAATAAATTATATAATGACCTTTGTGTGAAATAAGGATCTT CATCCTTAACATATTTTTTGGTGGTGAACTACTGTGGAAGTTACTGACGTA AGATTACGG |
| 30 | GATCTTTAGTTTGTTCTACCATGTTTTTATCACCTAAAAGTTTACCACTAA TTTTTGTTTATTATATCATAAACGGTGAAGCAATAATGGAGGAATGGTTG ACTTCAAAACAAATAAATTATATAATGACCTTTGTGTGAAATGGAGCCGC TGAGCTACCACAGATTGTGAAAGGAGAGGTTAAC |
| 31 | GATCTTTAGTTTGTTCTACCATGTTTTTATCACCTAAAAGTTTACCACTAA TTTTTGTTTATTATATCATAAACGGTGAAGCAATAATGGAGGAATGGTTG ACTTCAAAACAAATAAATTATATAATGACCTTTGTGTGAAATAAAGGAGG AATTCAAAATGGAAGTTACTGACGTAAGATTACGG |
| 32 | GATCTTTAGTTTGTTCTACCATGTTTTTATCACCTAAAAGTTTACCACTAA TTTTTGTTTATTATATCATAAACGGTGAAGCAATAATGGAGGAATGGTTG ACTTCAAAACAAATAAATTATATAATGACCTTTGTGTGAAAT |
| 33 | GTGTCAAAACGCATACCATTTTGAACGAGTTGGCACAGTGAAAGCCG |
| 34 | CTATTCCTTTGTCGGTTTTGCCG |
| 35 | GATCTCGACCTGCAGCCCAAGCGAAATAAACTTACAATTTGAGAAAAAC |
| 36 | ACATATTCCCGTTATGCATCG |
| 37 | GCTTGGGCTGCAGGTCGAGATC |
| 38 | GTTCAAAATGGTATGCGTTTTGACAC |
| 39 | TAAAAATTTTACAAAAAGGTATTGACTTTCCCTACAGGGTGTGTAATAAT TTAATTGACGGTAAATAACAAAAGAGG |
| 40 | CAATACCTTTTTGTAAAATTTTTAGAAATAAACTTACAATTTGAGAAAAAC |
| 41 | CAGAGTAGACTTACTTAAAAGACTATTCTGCAATCTTTATTCATTTGTCC TTATAAG |
| 42 | TTTTAAGTAAGTCTACTCTGAATTTTTTTAGACGGTAAATAACAAAAGAG GGG |
| 43 | GTGTAAGGAGAAGGACAGGTGCTGCCCTTCGATAAAATCAATCTTTATT CATTTGTCCTTATAAG |
| 44 | CCTGTCCTTCTCCTTACACTTTGAGGGAGGTGAACACAGACGGTAAATA ACAAAAGAGGGG |

### Example 1: Integration of the aprE, grpE, cotG, SP82, RSBgsiB mRNA stabilizing element downstream of the native promoter of the riboflavin (rib) operon and the native strong promoter of rpsD

Protein overproduction mediated by the aprE, grpE, cotG, SP82 or RSBgsiB mRNA stabilizing element was tested via insertion of said mRNA stabilizing elements (SEQ ID NO:1 to 5) between the native *rib* operon promoter (*P_{rib}*) and the *lacZ* reporter gene in the first set of constructs. In a second set of constructs said mRNA stabilizing elements were inserted between the native strong rpsD promoter (*P_{rpsD}*) and the *lacZ* reporter gene.

First, the pBest4 vector was constructed carrying the *lacZ* reporter gene and multiple cloning sites (MCS): The pBestMCS+ (SEQ ID NO:21) and pBestMCS- (SEQ ID NO:22) oligonucleotides containing an *Asc*I restriction site were annealed and cloned into the *Hin*dIII and *Bam*HI digested pDG1728 plasmid (ECE114; Guerout-Fleury et al., 1996, Gene 180:57-61; available from BGSC). The resulting plasmid pBest4 was used to accommodate ten synthetic constructs at the 5'-end of the *lacZ* reporter gene, respectively.

In the first set of constructs with the native *P_{rib}* promoter, the four synthetic DNA fragments contained (i) the *rib* promoter and the *aprE* stabilizing element (P_{ribΩaprE}; SEQ ID NO:23); (ii) the *rib* promoter and the *grpE* stabilizing element (P_{ribΩgrpE}; SEQ ID NO:24); (iii) the *rib* promoter and the *cotG* stabilizing element (P_{ribΩcotG}; SEQ ID NO:25); and (iv) only the native *rib* promoter (P_{rib}; SEQ ID NO:26) used as a control.

In the second set of constructs with the native strong P_{rpsD} promoter the six synthetic DNA fragments contained (i) the rpsD promoter and the aprE stabilizing element (P_{rpsDΩaprE}; SEQ ID NO:27); (ii) the rpsD promoter and the grpE stabilizing element (P_{rpsDΩgrpE}; SEQ ID NO:28); (iii) the rpsD promoter and the cotG stabilizing element (P_{rpsDΩcotG}; SEQ ID NO:29); (iv) the rpsD promoter and the SP82 stabilizing element (P_{rpsDΩSP82}; SEQ ID NO:30); (v) the rpsD promoter and the gsiB stabilizing element [RBSgsiB] (P_{rpsDΩRBSgsiB}; SEQ ID NO:31); and (vi) only the native rpsD promoter (P_{rpsD}; SEQ ID NO:32) used as a control. In the synthetic DNA constructs (see above) the mRNA stabilizing elements were inserted 10 nucleotides downstream of the +1 transcription start site.

The synthetic DNA fragments were synthesized and custom cloned by DNA2.0 Inc. (Menlo Park, California, USA). The synthetic DNA fragments shown in SEQ ID NOs; 23, 25, 27, 29 and 31 were inserted between the *Bam*HI and SalI sites in pBest4 resulting in plasmids pBest55, pBest59, pBest70, pBest74, and pBest79, respectively. The synthetic DNA fragments shown in SEQ ID NOs: 26, 24, 32, 28 and 30 were inserted between the AscI and *Bam*HI sites in pBest4 resulting in plasmids pBest51, pBest57, pBest66, pBest72, and pBest77, respectively. The ten plasmids were transformed into *B. subtilis* 1A747 [SPβ^{c}, prototroph, derivative of *B. subtilis* 168 *(trpC2);* available from BGSC] selecting for spectinomycin resistance (Spec¹) to integrate the different *lacZ* fusions into the *amyE* chromosomal locus, generating strains BE111 *(P_{rib}-lacZ),* BE115 (P_{nbΩaprE}-lacZ), BE117 (P_{ribΩgrpE}-lacZ), BE119 (P_{nbΩ00tG}-lacZ), BE126 (P_{rpsD}-lacZ), BE130 (P_{rpsDΩaprE}-lacZ), BE132 (P_{rpsDΩgrpE}-lacZ), BE134 (P_{rpsDΩcotG}-lacZ), BE137 (P_{rpsDΩsp82}-lacZ) and BE139 (P_{rpsDΩRBSgsiB}-lacZ). The correct double cross-over integration was confirmed with the α-amylase assays (Pragai et al., 2001, J. Bacteriology 183:2505-2515): transformants with the derivatives of pBest4 plasmid were streaked onto TBAB agar (WO 04/106557) containing 1% starch (Sigma) for the test of the production of α-amylase. After 24 h of incubation at 37°C the halos produced as a result of starch hydrolysis were visualized with staining using Lugol (iodine/potassium iodide) solution (Sigma). After double cross-over recombination of the pBest4 derivatives into the *amyE* gene of the *B. subtilis* 1A747 chromosome, no halo was formed around the two BE strains carrying the different *lacZ* fusions and *spec* gene in the *amyE* gene colonies of the Spec^{r} transformants.

Determination of β-galactosidase activities were performed as follows: *B. subtilis* BE strains were inoculated in LB rich medium containing 100 µg/ml Spec and in BFA defined minimal medium containing 100 µg/ml Spec and grown overnight at 37°C with an agitation of 250 rpm. Overnight cultures were diluted 100-fold in fresh LB medium (25 g LB broth, Merck, Catalog # 1.10285.0500 ad 11 H₂O, autoclaved) and in fresh BFA defined minimal medium (1X Minimal Salt Solution, supra; 1X Trace elements solution B, supra; 0.4 % glucose; 0.2 % L-glutamine; 4 mg FeCl₃; 0.2 mg MnSO₄, ad 11 H₂O) and grown at 37°C with an agitation of 250 rpm. Samples were collected hourly for determination of optical density at 600 nm (OD₆₀₀) and β-galactosidase activity (cell pellet from 0.1-1 ml of culture). The β-galactosidase samples were stored at -20°C. The β-galactosidase assay was performed and the specific β-galactosidase activity (expressed in nmol ONP/min/OD₆₀₀; ONP is 2-nitrophenol) was determined as described previously (Prágai and Harwood, 2002, Microbiology 148:1593-1602). As a result, BE115 strain produced 4-5-fold more specific β-galactosidase activity, BE117 produced 2-2.5-fold more specific β-galactosidase activity and BE119 produced 2-2.5-fold more specific β-galactosidase activity than the isogenic strain BE111 (P_{rib}-lacZ) without the mRNA stabilizing elements in both tested media. When using the rpsD promoter strains the results were as follows: BE 132 strain produced 2.5-3-fold more specific β-galactosidase activity, BE134 produced 2-2.5-fold more specific β-galactosidase activity, BE137 produced 1.5-2-fold more specific β-galactosidase activity and BE139 produced 17-18-fold more specific β-galactosidase activity than the isogenic strain BE126(P_{rpsD}-lacZ) without the mRNA stabilizing elements in both tested media.

### Example 2: Production of riboflavin via deregulation and use of the mRNA stabilizing elements

To analyse the effect of the mRNA stabilizing elements on riboflavin production in *Bacillus subtilis,* two mRNA stabilizing elements, i.e. SEQ ID NO:1 and 2 were inserted upstream of the *rib* operon. Four strains are constructed carrying (i) the wild-type *P_{rib}* promoter [control] (ii) the *P₁₅* constitutive strong promoter [deregulation]; and (iii) the *P₁₅* constitutive strong promoter and the respective mRNA stabilizing elements [deregulation and mRNA stabilization]. The *P₁₅* promoter of the *B. subtilis* bacteriophage SPOI (Lee et al., 1980, Mol. Gen. Genet. 180:57-65) was obtained from plasmid pXI23roDTD-SPO1-15, a derivative of plasmid pX12 (Hümbelin et al., 1999, J. Ind. Microbiol. Biotech. 22:1-7).

For replacing the native *P_{rib}* promoter with *P₁₅* constitutive strong promoter and inserting mRNA stabilizing elements upstream of the five *rib* genes, first, the riboflavin-auxotroph *B. subtilis* BS3813 strain was constructed. In BS3813, the promoter of the riboflavin operon, the 5' untranslated sequence and the 5' end of *ribD* structural gene was replaced by a neomycin resistance *(neo)* gene obtained from plasmid pUB110 (Itaya et al., 1989, Nucleic Acid Res. 17:4410). For the strain construction, Long Flanking Homology Polymerase Chain Reaction (LFH-PCR) was used to generate DNA fragments containing the 1236-bp *neo* gene flanked with the 526-bp upstream region of the native *P_{rib}* promoter (flank 5') and the 502-bp 3' end of *ribD* gene (flank 3'). Therefore, 3 DNA fragments flank 5', the *neo* gene and flank 3' were first PCR amplified. Generating DNA fragments flank 5' and flank 3' were generated as follows: 0.2 µl of a 100 mM solution of primers p50 (SEQ ID NO:33) together with p51 (SEQ ID NO:34) or primers p44 (SEQ ID NO:35) together with p45 (SEQ ID NO:36) were added to 0.1 µg *B. subtilis* 1A747 chromosomal DNA in a 50 µl reaction volume containing 1 µl of 40 mM dNTP's, 5 µl of 10X buffer and 0.5 µl Pfu polymerase enzyme (Stratagene). For generating DNA fragment containing the *neo* gene, 0.2 µl of a 100 mM solution of primers p9 (SEQ ID NO:37) together with p10 (SEQ ID NO:38) were added to 0.05 µg pUB 110 DNA containing the *neo* gene in a 50 µl reaction volume containing 1 µl of 40 mM dNTP's, 5 µl of 10X buffer and 0.5 µl Pfu polymerase enzyme (Stratagene). The PCR reactions were performed in 30 cycles of three sequential steps: (i) denaturing step at 95°C for 30 sec; (ii) annealing step at 52°C for 30 sec; (iii) elongation step at 72°C for 1.5 min. The 3 PCR products were separated by agarose gel electrophoresis and extracted from the gel using the MinElute Gel Extraction Kit (Qiagen). In the final LFH-PCR reaction, the 3 purified PCR products (flank 5', neo gene and flank 3') were assembled: 0.2 µl of a 100 mM solution of primers p45 together with p51, 0.5 µl flank 5' PCR product (25 ng), 0.5 µl flank 3' PCR product (25 ng) and 2 µl neo resistance gene (100 ng) were added in a final 50 µl reaction volume containing 1 µl of 40 mM dNTP's, 5 µl of 10X buffer and 0.5 µl HF Expand polymerase enzyme (Roche Biochemicals). The LFH-PCR reaction was performed in 35 cycles of three sequential steps: (i) denaturing step at 95°C for 30 sec; (ii) annealing step at 52°C for 30 sec; (iii) elongation step at 72°C for 2.5 min. The assembled LFH-PCR product was purified by using the QiaQuick PCR purification kit (Qiagen). The purified LFH-PCR product (2 µg) was used for competent cell transformation of *B. subtilis* 1A747. Neomycin-resistant (Nm^{r}) transformants were selected on TBAB plates containing 2 mg/L neomycin and 100 mg/L riboflavin. The correct genotype of the resulting riboflavin-auxotroph and Nm^{r} BS3813 strain was confirmed by two PCR reactions using primers p45 together with p10, and primers p51 together with p9, and chromosomal DNA of the transformants as template DNA. The PCR reaction and protocol were performed using standard reaction conditions as described above for the generation of DNA fragments containing flank 5' and flank 3'. In addition, the sequence of the 3' end of *ribD* in BS3813 was confirmed by sequencing.

In the second strain construction step, the *neo* gene in the riboflavin-auxotroph *B. subtilis* BS3813 strain is replaced using two LFH-PCR products containing (i) the *P₁₅* constitutive strong promoter (P₁₅); and (ii) the *P₁₅* constitutive strong promoter and the respective mRNA stabilizing elements upstream of the *rib* operon.

For the construction of LFH-PCR product containing *P₁₅ribDEAHT* construct two standard PCR reactions were performed in which the sequence of *P₁₅* promoter (Lee et al., 1980, Mol. Gen. Genet 180:57-65) was introduced into the PCR products. In a third PCR reaction the two PCR products were combined. For amplifying PCR product 1 containing the 5' region of the riboflavin operon at the 5'-end of the *P₁₅* promoter, the primer pair p45 and Spo15S' (SEQ ID NO:39) and the chromosomal DNA from strain *B. subtilis* 1A747 as template was used under standard PCR conditions. For amplifying PCR product 2 containing the *ribD* at the 3'-end of the *P₁₅* promoter, primers p51 together with p63 (SEQ ID NO:40) and the chromosomal DNA from strain *B. subtilis* 1A747 as template was used under standard PCR conditions. In the standard LFH-PCR reaction, the gel-purified PCR products 1 and 2 were assembled into one DNA fragment as described before. The purified LFH-PCR product was transformed into the riboflavin-auxotroph *B. subtilis* BS3813 competent cells, in which the riboflavin promoter region and the 5' part of *ribD* was replaced with neo gene. Riboflavin-prototroph transformants were selected on roseoflavin selection minimal agar medium (RSMM: 880 ml 1.5 % agar in distilled H₂O; autoclaved for 30 min at 121°C followed by addition of 100ml 10X Spizizen salts, supra, 4 ml 50% glucose, 100 mg roseoflavin, 10 ml 100X Trace elements solution A, supra) containing 100 mg/L roseoflavin, a riboflavin anti-metabolite that prevents the growth of cross-fed non-transformant recipient cells. The riboflavin-prototroph *Bacillus* transformants were suspended in 1 ml 0.9% NaCl solution and 100 µL the 500-fold dilution of the original cell suspension was plated on TBAB agar plate. Single colonies were transferred onto fresh TBAB agar plates and TBAB agar plates supplemented with 2 mg/L Nm and 100 mg/L riboflavin. The correct transformants grew only on TBAB agar plate and therefore they were neomycin-sensitive. In addition, the genotype was confirmed with sequencing of the *ribD* region. The final strain was called BS3944 (P₁₅ribDEAHT).

For the construction LFH-PCR product containing P_{15ΩaprE} construct two standard PCR reactions were performed in which the DNA sequence of the aprE mRNA stabilizing element was integrated into two PCR products. For amplifying PCR product 1 containing the 5' region of the riboflavin operon at the 5'-end of the aprE mRNA stabilizing element, the primers p45 together with p143 (SEQ ID NO:41) and the chromosomal DNA from strain BS3944 as template was used under standard PCR conditions. For amplifying PCR product 2 containing the *ribD* at the 3'-end of the aprE mRNA stabilizing element, the primers p51 together with p142 (SEQ ID NO:42) and the chromosomal DNA from strain BS3944 as template were used under standard PCR conditions. In the standard LFH-PCR reaction, the gel-purified PCR products 1 and 2 were assembled into one DNA fragment as described before. The purified LFH-PCR product was transformed again into the riboflavin-auxotroph *B. subtilis* BS3813 competent cells, in which the riboflavin promoter region and the 5' part of *ribD* was replaced with neo gene. Riboflavin-prototroph transformants were selected on RSMM plates. The riboflavin-prototroph *Bacillus* transformants were suspended in 1 m1 0.9% NaCl solution and 100 µL the 500-fold dilution of the original cell suspension is plated on TBAB agar plate. Single colonies were transferred onto fresh TBAB agar plates and TBAB agar plates supplemented with 2 mg/L Nm and 100 mg/L riboflavin, The correct transformants grew only on TBAB agar plate and therefore they are neomycin-sensitive. In addition, the genotype was confirmed with sequencing of the *ribD* region. The resulting strain was BS5193 (P_{15ΩaprE}ribDEAHT).

The same method was applied with the grpE mRNA stabilizing element resulting is BS5196 (P_{15ΩgrpE}ribDEAHT) using primer pair p145 (SEQ ID NO:43) and p144 (SEQ ID NO:44).

In order to transfer the new constructs into a riboflavin producing background, three PBS1 phage lysates from the BS3944, BS5193 and BS5196 were prepared as described in Cutting and Vander Horn, 1990, Genetic Analysis in Harwood and Cutting (eds), Molecular biological methods for Bacillus. New York: John Wiley and Sons. Generalized transduction mediated by PBS1 phage was used to transfer the two constructs P₁₅ribDEAHT, P_{15ΩaprE}ribDEAHT and P_{15ΩgrpE}ribDEAHT into the BSS178 (ΔribD::neo tktR357A, ribC, spo0A12) strain. The BS5178 strain is riboflavin-auxotroph since the promoter of the riboflavin operon, the 5' untranslated sequence and the 5' end of *ribD* structural gene was replaced by a neo gene similarly as it was described before for BS3813. After the PBS 1 mediated transduction of BS5178, riboflavin-prototroph and Nm sensitive strains were selected on RSMM plates as described before. The resulting strains were BS5240 (P₁₅ribDEAHT), BS5237 (P_{15ΩaprE}ribDEAHT) and BS5238 (P_{15ΩgrpE}ribDEAHT). The control isogenic strain with the wild-type *rib* operon was B55191 (P_{rib}ribDEAHT). The four strains BS5191, BS5240, BS5137 and BS5138 were evaluated for riboflavin production in shake flask cultures: Strains were inoculated from frozen glycerol stocks in 5 ml VY rich medium (WO 04/106557) and grown overnight at 37°C with an agitation of 280 rpm. Cells were collected by centrifugation and resuspended in 1 ml riboflavin screening medium (RSM: 100ml 10X Spizizen salts, supra; 10 ml 100X Trace elements solution A, supra, 2 ml 50% glucose; 36 ml 25% raffinose 10 ml 10% yeast extract, ad 11 H₂O). 250 µl of the cell suspension is used for inoculation of 25 ml RSM in 250 mL baffled shake flasks. After 48 h incubation at 39°C with an agitation of 220 rpm, 500 µl culture were taken and 35 µl 4N NaOH was mixed with the sample for 1 minute at room temperature allowing to dissolve the riboflavin crystals. Samples were neutralized by the addition of 465 µl 1 M potassium phosphate buffer (pH 6.8) and pelleted with centrifugation (5 min, 13.2 krpm). The supernatant was used for HPLC determination of the concentrations of riboflavin and two side products: 6,7-dimethyl-8-ribityllumazine (DMRL) and oxolumazine. In addition, a second culture sample was taken and after centrifugation (5 min, 13.2 krpm) the supernatant was used for the determination of the concentrations of the residual glucose and raffinose in the medium.

HPLC analysis for riboflavin analysis was performed as follows: samples from shake flask cultures were analyzed by HPLC using an Agilent 1100 HPLC system equipped with a thermostatted autosampler, a diode array and a fluorescence detector. The separation was performed on a Supelcosil LC-8DB-5µ column (150 mm x 4.6 mm) equipped with a 4mm LC-8DB guard column. A mixture of 0.1M acetic acid and methanol was used as mobile phase. Gradient elution was applied starting at 2% methanol (constant for 5 min) and going up to 50% methanol in 15 minutes. The column was kept at 20°C. The signal was recorded by UV at 280 nm. Riboflavin was well separated from the impurities (*e.g.* side products: DMRL and oxolumazine) and eluted at 15.2 minutes. The calibration was based on reference material obtained from Fluka. The calibration range of the method was from 10µg/ml to 1 mg/ml. For HPLC analysis of the sugars, the following procedure was applied using an Agilent 1100 series HPLC system with a quaternary pump, an autosampler a UV- and a refractive index detector. The separation was achieved on a CAPCELL PAK NH2 UG80 column (4.6 mm x 250 mm, 5µ) (Shiseido). The optimal column temperature was 35°C. The mobile phase was a mixture of acetonitrile and DI water at a 65/35 ratio. The flow rate was 1.0 ml/min and the injection volume set to 5 µl. The refractive index signal was monitored and used for detection. The calibration range for each compound was from 0.5 mg/ml to 30 mg/ml.

The results show that increasing the transcription level of the *ribDEAHT* operon by the constitutive strong *P₁₅* promoter and deleting the regulatory riboswitch sequence [deregulation] results in 2.3-fold higher riboflavin yield on carbon source for BS5240 than obtained with the control strain BS5991 containing the wild-type *rib* operon. Using the constitutive strong *P₁₅* promoter and increasing the stability of the *ribDEAHT* transcripts by the aprE and grpE mRNA stabilizing element [deregulation and mRNA stabilization], respectively led to a 3.9-fold and 6.4-fold, respectively higher riboflavin yield on carbon source for BS5237 and BS5238, respectively than it was produced by the control strain BS5191, thus an increase of at least 50%. A comparison between the amount of riboflavin produced from the control strain BS5191 containing the wild-type *rib* operon and a strain containing the natural P*_{rib}* together with the respective mRNA stabilizing elements shows an increase of at least 10% when using the mRNA stabilizing elements. The results show that mRNA stabilization is a powerful tool for strain engineering and it enhances significantly the increase of riboflavin yield resulted both using the natural promoter as well as by the deregulation of the *rib* operon with the constitutive strong SPO1 promoter *P₁₅.*

### Example 3: Integration of the mRNA stabilizing elements downstream of the genes

### involved in biotin, pantothenic acid and α-amylase

The mRNA stabilizing elements can be used to increase the yield of a target fermentation product other than riboflavin, such as e.g. biotin, pantothenic acid or α-amylase. Construction of genetically engineered *B. subtilis* strains carrying the respective mRNA stabilizing elements downstream of either the natural promoter or a strong constitutive promoter such as e.g. P*₁₅* is performed according to Example 2 or Figure 1.

The amount of the respective target fermentation product is increased by at least 10% when using the natural promoter and at least 50% when using the strong constitutive promoter.

## Claims

1. A polynucleotide selected from the group consisting of:
(a) polynucleotides comprising the nucleotide sequence according to SEQ ID NO:1 to 5;
(b) polynucleotides comprising a nucleotide sequence obtainable by nucleic acid amplification such as polymerase chain reaction, using genomic DNA from a microorganism as a template and a primer set according to SEQ ID NO:6 / SEQ ID NO:7, SEQ ID NO:8 / SEQ ID NO:9, SEQ ID NO;10 / SEQ ID NO:11, SEQ ID NO:12 / SEQ ID NO:13 and SEQ ID NO:14 SEQ ID NO:15, respectively;
(c) polynucleotides comprising a nucleotide sequence which is a fragment or derivative of a polynucleotide according to (a) or (b), said fragment or derivative having the activity of an mRNA stabilizing element;
(d) polynucleotides the complementary strand of which hybridizes under stringent conditions to a polynucleotide as defined in any one of (a) to (c) and which have the activity of an mRNA stabilizing element; and
(e) polynucleotides which are at least 60%, such as 70, 85, 90 or 95% identical to a polynucleotide as defined in any one of (a) to (c) and which have the activity of an mRNA stabilizing element
or
the complementary strand of such a polynucleotide.

2. A process for the production of a target fermentation product with a microorganism wherein said microorganism is incubated in a aqueous medium under conditions that allow the production of said target fermentation product from a carbon source and wherein optionally the target fermentation product is isolated as the fermentation product, wherein said microorganism is genetically altered by introduction of a polynucleotide according to claim 1 that it leads to an improved yield and/or efficiency of production of the target fermentation product produced by said microorganism.

3. A process for the production of mRNA with increased stability transcribed from a gene involved in the biosynthetic pathway of a target fermentation product in a microorganism, said microorganism comprising a polynucleotide according to claim 1 introduced downstream of the transcription start of the respective gene(s),

4. A process for the production of a microorganism capable of producing a target fermentation product, said process comprising the step of altering said microorganism by introduction of a polynucleotide according to claim 1 so that the microorganism produces a stabilized mRNA leading to an improved yield and/or efficiency of production of the target fermentation product produced by said microorganism.

5. A process for the production of a microorganism wherein the stability of mRNA transcribed from an endogenous gene is increased, comprising the step of altering said microorganism by introduction of a polynucleotide according to claim 1 downstream of the transcription start of the respective endogenous gene.

6. The process according to any one of claims 2 to 4, wherein the target fermentation product is selected from vitamins or enzymes.

7. The process according to claim 6, wherein the target fermentation product is selected from the group consisting of riboflavin, biotin, pantothenic acid, folic acid, thiamin, pyridoxin, transferases [EC 2], and glycosylases [EC 3].

8. The process according to any one of claims 2 to 7, wherein the microorganism is selected from the group consisting of *Escherichia, Gluconobacter, Rhodobacter, Pseudomonas, Paracoccus, Bacillus, Brevidbacterium, Corynebacterium, Rhizobium* (*Sinorhizobium*), *Flavobacterium, Klebsiella, Enterobacter, Lactobacillus, Lactococcus* and *Streptomyces,* preferably *Bacillus,* more preferably *Bacillus subtilis.*

9. A microorganism produced by a process according to any one of claims 4, 5 or 8.

10. A microorganism genetically engineered with a polynucleotide according to claim 1.

11. The microorganism according to claim 10, said microorganism being genetically altered in such a way that it leads to an improved yield and/or efficiency of production of a target fermentation product produced by said microorganism.

12. The microorganism according to claim 10 producing an mRNA transcript with increased and/or improved stability,

13. The microorganism according to any one of claims 9 to 12 which is selected from the group consisting of *Escherichia, Gluconobacter, Rhodobacter, Pseudomonas, Paracoccus, Bacillus, Brevibacterium, Corynebacterium, Rhizobium (Sinorhizobium), Flavobacterium, Klebsiella, Enterobacter, Lactobacillus, Lactococcus* and *Streptomyces,* preferably *Bacillus,* more preferably *Bacillus subtilis.*

14. The microorganism according to claim 11 or 13, wherein the target fermentation product is selected from vitamins or enzymes.

15. The microorganism according to claim 14, wherein the target fermentation product is selected from the group consisting of riboflavin, biotin, pantothenic acid, folic acid, thiamin, pyridoxin, transferases [EC 2], and glycosylases [EC 3],
